# EUROPÄISCHE PATENTSCHRIFT

(11) **EP 1 965 915 B1**
(45) Veröffentlichungstag und Bekanntmachung des Hinweises auf die Patenterteilung: **13.08.2014**
(21) Anmeldenummer: 06830411.2
(22) Anmeldetag: 06.12.2006
(51) Int. Cl.: B01J 31/16, B01J 31/22, C07C 67/333, C07C 41/18, C07F 15/00, C07B 37/10, B01J 31/06

(54) **GETRÄGERTER ÜBERGANGSMETALLKOMPLEX UND DESSEN VERWENDUNG IN DER KATALYSE**
SUPPORTED TRANSITION METAL COMPLEX AND USE THEREOF IN CATALYSIS
COMPLEXE DE METAUX DE TRANSITION SUPPORTE ET SON UTILISATION EN CATALYSE

(30) Priorität: 06.12.2005 DE 102005058255; 09.12.2005 DE 102005058980
(43) Veröffentlichungstag der Anmeldung: 10.09.2008
(73) Patentinhaber: Rapp Polymere GmbH, 72072 Tuebingen (DE)
(72) Erfinder: BANNWARTH, Willi, 79104 Freiburg-im-Breisgau (DE); MICHALEK, Florian, 79108 Freiburg-im-Breisgau (DE); RÜHE, Jürgen, 79356 Eichstetten (DE)
(74) Vertreter: Reitstötter Kinzebach
(86) Internationale Anmeldenummer: PCT/EP2006/069365
(87) Internationale Veröffentlichungsnummer: WO 2007/065907

(56) Entgegenhaltungen:
- FIKRET KOC ET AL: "Catalysts on functionalized polymer chips (PC) as recyclable entities" SYNTHESIS, Nr. 19, 14. November 2005 (2005-11-14), Seiten 3362-3372, XP002421138 in der Anmeldung erwähnt
- BUCHMEISER M R: "Recent advances in the synthesis of supported metathesis catalysts" NEW JOURNAL OF CHEMISTRY, CNRS-GAUTHIER-VILLARS, MONTROUGE, FR, Bd. 28, Nr. 5, 8. April 2004 (2004-04-08), Seiten 549-557, XP002372864 ISSN: 1144-0546 in der Anmeldung erwähnt
- FUERSTNER A ET AL: "Olefin Metathesis in Supercritical Carbon Dioxide" JOURNAL OF THE AMERICAN CHEMICAL SOCIETY, AMERICAN CHEMICAL SOCIETY, WASHINGTON, DC, US, Bd. 123, Nr. 37, 2001, Seiten 9000-9006, XP002331227 ISSN: 0002-7863 in der Anmeldung erwähnt
- FLORIAN MICHALEK ET AL: "Ring-closure metathesis in supercritical carbon dioxide as sole solvent with use of covalently immobilized Ruthenium catalysts" EUR. J. ORG. CHEM., Bd. 2006, Nr. 3, 13. Dezember 2005 (2005-12-13), Seiten 577-581, XP002421139
- FLORIAN MICHALEK ETA L: "The activity of covalently immobilized Grubbs-Hoveyda type catalyst is highly dependent on the nature of the support material" JOURNAL OF ORGANOMETALLIC CHEMISTRY, Bd. 691, 30. Juni 2006 (2006-06-30), Seiten 5172-5180, XP002421140

## Beschreibung

Die vorliegende Erfindung betrifft Olefinmetathese-Katalysatoren in Form eines geträgerten Übergangsmetallkomplexes und deren Verwendung in der Olefinmetathese sowie entsprechende Verfahren zur übergangsmetallkatalysierten Olefinmetathese .

Die einfache Abtrennbarkeit und Wiederverwendung von Katalysatoren sind sowohl für industrielle Verfahren als auch für akademische Fragestellungen von herausragender Bedeutung. Unter ökonomischen Aspekten sollte ein Katalysator idealerweise vollständig wiederverwertbar sein. Diesbezüglich weisen immobilisierte und heterogene Katalysatoren gegenüber homogenen praktische und ökonomische Vorteile auf.

Allerdings ist die Auswahl eines geeigneten Trägers und die Art der Anbindung des Katalysators an den Träger mit einigen Schwierigkeiten verbunden. Insbesondere gilt es zu beachten, dass die mit homogenen Übergangsmetallkatalysatoren erzielbaren hohen Aktivitäten, Selektivitäten und Reaktionsraten erhalten bleiben, und die Produkte möglichst frei von Metall- und sonstigen Verunreinigungen sind.

Auf dem Gebiet der Olefinmetathese beispielsweise waren erste Versuche zur Anbindung von Katalysatoren, die auf Rutheniumalkylidenen vom Grubbs-Typ basierten, an Polystyrol nur begrenzt erfolgreich, und der entstandene Polymer-gebundene Katalysator war ungefähr zwei Größenordnungen weniger aktiv als das homogene Analogon. Weiterhin führten die Wiederaufarbeitung und Wiederverwendung zu Aktivitätsverlusten. Inzwischen gab es weitere Versuche, besagte Metathesekatalysatoren zu trägern. Eine Übersicht hierzu findet sich beispielsweise in M. R. Buchmeiser, New J. Chem. 2004, 28, 549-557.

Zahlreiche Vorteile werden mit der Verwendung von überkritischem Kohlendioxid als Reaktionsmedium für übergangsmetallmetallkatalysierte Reaktionen in Verbindung gebracht. Prozesstechnische Aspekte wie die Nichtbrennbarkeit, die ökologische und toxikologische Unbedenklichkeit, das prinzipielle Vermeiden einer Gas/Flüssig-Phasengrenze sowie mögliche Vereinfachungen in der Aufarbeitung sprechen für die Verwendung von überkritischem Kohlendioxid als Alternative zu konventionellen Lösungsmitteln. So wurde bereits am Beispiel der Olefinmetathese beschrieben, dass es nicht nur möglich ist, bekannte Reaktionen in das überkritische Medium zu "übertragen", sondern dass es das vorteilhafte Eigenschaftsprofil von Kohlendioxid erlaubt, die Anwendungsbreite einer etablierten Reaktion in bemerkenswerter Weise zu erweitern (A. Fürstner, D. Koch, K. Langemann, W. Leitner, C. Six, Angew. Chem. 1997, 109, 2562-2565; Angew. Chem. Int. Ed. Engl. 1997, 36, 2466-1469; A. Fürstner. L. Ackermann, K. Beck, H. Hori, D. Koch, K. Langemann, M. Liebl, C. Six, W. Leitner, J. Am. Chem. Soc. 2001, 123, 9000-9006.

Die homogene Katalyse in überkritischem Kohlendioxid ist allerdings oftmals durch die geringe Löslichkeit des Katalysators in dem unpolaren Lösungsmittel beeinträchtigt. Dies wird derzeit häufig dadurch umgangen, dass man die Ligandensphäre des Katalysators mit perfluorierten Gruppen modifiziert, um die Löslichkeit zu erhöhen. Ein Beispiel hierfür ist die Stille-Kopplung in überkritischem Kohlendioxid mit Pd-Komplexen, deren Phosphinliganden perfluorierte Substituenten aufweisen (T. Osswald, S. Schneider, S. Wang, W. Bannwarth, Tetrahedron Lett. 2001, 42, 2965-2967). Während die Einführung perfluorierte Substituenten zwar zu einer erhöhten Löslichkeit des Katalysators in überkritischem Kohlendioxid beiträgt, ist damit allerdings auch die Möglichkeit eines verstärkten Ausblutens (sogenanntes "leaching") des Katalysators verbunden.

Davon ausgehend war es Aufgabe der vorliegenden Erfindung, Katalysatoren zur Verfügung zu stellen, deren Anwendung in überkritischem Kohlendioxid bei hohen Umsätzen mit einem vergleichsweise geringen Ausbluten einhergeht.

Überraschenderweise wurde nun gefunden, dass obige Aufgabe dadurch gelöst wird, dass man Übergangsmetallkomplexe an spezielle Träger bindet.

Gegenstand der vorliegenden Erfindung sind daher die in den Patentansprüchen 1 und 3 offenbarten geträgerten Übergangsmetallkomplexe.

Gegenstand der vorliegenden Erfindung ist auch die Verwendung der erfindungsgemäßen geträgerten Übergangsmetallkomplexe in der Olefinmetathese.

Des weiteren wird ein Verfahren zur übergangsmetallkatalysierten Olefinmetathese in überkritischem Kohlendioxid offenbart, das dadurch gekennzeichnet ist, dass der Olefinmetathese-Katalysator ein nachstehend offenbarter geträgerter Übergangsmetallkomplex ist.

Als Träger eignen sich bestimmte sowohl organische als auch anorganische Materialien, die in überkritischem Kohlendioxid im Wesentlichen unlöslich sind. Allerdings können insbesondere die organischen Träger in überkritischem Kohlendioxid quellbar sein.

Die Art der Anbindung des Übergangsmetallkomplexes über die Gruppe X richtet sich nach der Art des Komplexes und insbesondere die von diesem zwecks Anbindung bereitgestellten funktionellen Gruppen. In der Regel wird man Ether-, Ester-, Amid- oder Urethanbindungen wegen ihrer leichten synthetischen Zugänglichkeit bevorzugen, soweit diese eine stabile Anbindung des Übergangsmetallkomplexes unter den Anwendungsbedingungen gewährleisten.

Unter den Polystyrolen finden sich geeignete Pofymer-Matrices, die auch als Merrifield-Harze bezeichnet werden, von denen insbesondere die z.B. mit Hilfe von Divinylbenzol quervernetzten Polystyrole zu nennen sind. Erfindungsgemäß von besonderer Bedeutung sind Polystyrole mit einem relativ geringen Quervernetzungsgrad, beispielsweise solchen, die durch Quervernetzung mit 0,5 bis 2 %, vorzugsweise etwa 1 % Divinylbenzol (in mol-% bezogen auf die in der Polymerisation eingesetzten Monomere) erhältlich sind.

Einer Ausführungsform der vorliegenden Erfindung zufolge weist der Olefinmetathese-Katalysator in Form eines geträgerten Übergangsmetallkomplexes eine Polystyrol-Matrix auf, die Struktureinheiten der Formel (Ia) umfasst: 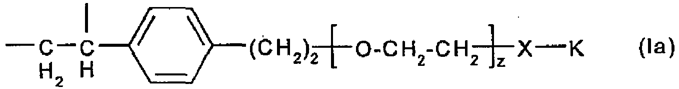 worin
- z: den Wert einer Zahl von 3 bis 20 hat;
- X: für eine direkte Bindung, Sauerstoff, Schwefel, -N(R¹)-, -C(=O)O-, -O(O=)C-, -N(R¹)(O=)C-, -C(=O)N(R¹)-, -O-CHR¹-O-, -OC(=O)N(R)-, - N(R¹)C(=O)O-, >C(=O) oder >C(=S) steht;
- R¹: für Wasserstoff oder C₁-C₄-Alkyl steht; und
- K: ein Wolfram-, Molybdän- oder Rutheniumkomplex ist.

Obigen Ausführungen entsprechend steht der die mittlere Anzahl von Polyethylen-Einheiten pro Polyethylen-Oligomer angebende Index z in der Formel (la) vorzugsweise für eine Zahl mit einem Wert im Bereich von 5 bis 15. Erfindungsgemäß besonders bevorzugt sind geträgerte Übergangsmetallkomplexe der Formel (Ia), worin z etwa den Wert 10 besitzt.

Die erfindungsgemäßen Polymer-Matrices mit daran gebundenen Ethylenglykol-Oligomeren, beispielsweise Polystyrol-Matrices, die Struktureinheiten der Formel (VI) umfassen: 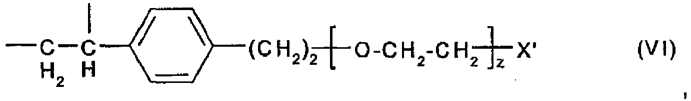 worin X' für eine Gruppe steht, die durch Umsetzung mit einem entsprechend modifizierten Übergangsmetallkomplex die Gruppe X bildet, z.B. OH, SH und NH₂, sind beispielsweise durch Umsetzung von Hydroxyethyl-funktionalisiertem Polystyrol mit Ethylenoxid und erforderlichenfalls einer Überführung der terminalen Hydroxygruppen in die Gruppen X' erhältlich und können darüber hinaus kommerziell bezogen werden, beispielsweise als die unter dem Handelsnamen HypoGel® von Rapp Polymere GmbH, Tübingen (Deutschland) vetriebenen Produkte.

Einer weiteren Ausführungsform der Erfindung zufolge basiert der geträgerte Übergangsmetallkomplex auf einer Kieselgel-Matrix, an die Acrylamid-Styrol-Copolymer gebunden ist. Dabei dienen die Acrylamid-Einheiten sowohl zur Bindung an die Matrix als auch zur Anbindung des Übergangsmetallkomplexes. Demnach weisen die geträgerten Übergangsmetallkomplexe dieser Art Struktureinheiten der Formel (II) auf: 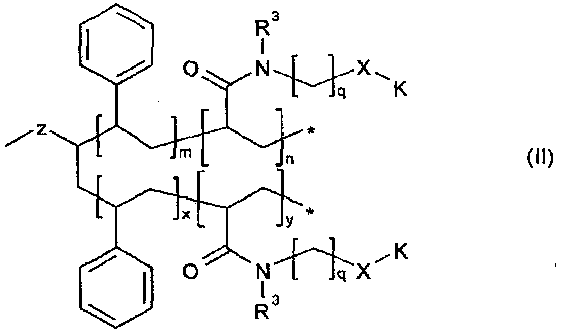 worin
- Z: für eine Bindung oder einen Spacer steht;
- m: den Wert einer Zahl von 1 bis 5000 hat;
- n: den Wert einer Zahl von 1 bis 5000 hat;
- x: den Wert einer Zahl von 1 bis 5000 hat;
- y: den Wert einer Zahl von 1 bis 5000 hat;
- X: für eine direkte Bindung, Sauerstoff, Schwefel, -N(R¹)-, -C(=O)O-, -O(O=)C-, -N(R¹)(O=)C-, -C(=O)N(R¹)-, -O-CHR¹-O-, -OC(=O)N(R¹)-, - N(R¹)C(=O)O-, >C(=O) oder >C(=S) steht;
- R¹: für Wasserstoff oder C₁-C₄-Alkyl steht;
- R³: für Wasserstoff oder C₁-C₄-Alkyl steht;
- q: den Wert einer Zahl von 2 bis 5 hat,
- K: ein Wolfram-, Molybdän- oder Rutheniumkomplex ist,
wobei die mit m, n, x und y indizierten Monomereinheiten statistisch verteilt sind.

Vorzugsweise liegt das Molverhältnis von (m + x) : (n + y), also das Molverhältnis von Styroleinheiten zu Acrylamideinheiten, im Bereich von 99:1 bis 60:40, insbesondere im Bereich von 98:2 bis 70:30 und besonders bevorzugt im Bereich 97:3 bis 75:25.

Kieselgel stellt unter den anorganischen Materialien die am häufigsten verwendete Matrix dar, da es neutral ist und seine Eigenschaften sowie die Möglichkeit zur Modifikation seiner Oberfläche gut untersucht sind. So lassen sich die Oberflächen solcher anorganischer Materialien in an sich bekannter Weise mit funktionellen Gruppen versehen, über die das Copolymer gebunden werden kann. Dazu verwendet man in der Regel Silane, insbesondere Alkoxysilane, die einerseits an freie Oberflächen-Hydroxygruppen binden, andererseits eine Funktionalität tragen, über die das Copolymer, direkt oder indirekt, an die Oberfläche der anorganischen Matrix gebunden werden kann. Insbesondere erlauben es an sich bekannte Sol-Gel-Verfahren, maßgeschneiderte Polysiloxane aufweisende Matrices mit geeigneten Funktionalitäten zur Verfügung zu stellen.

Gemäß einer bevorzugten Ausführungsform der vorliegenden Erfindung weist der Olefinmetathese-Katalysator in Form eines geträgerten Übergangsmetallkomplexes eine Kieselgel-Matrix auf, die Struktureinheiten der Formel (IIa) umfasst: 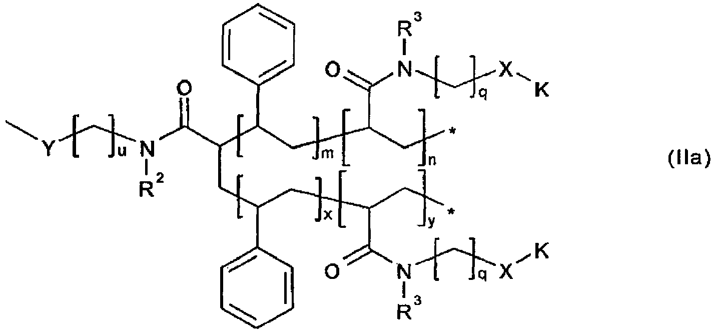 worin
- Y: für Si, Si(OR⁴) oder Si(OR⁴)₂ steht, wobei die freien Valenzen des Siliziums zum einen an das Alkylen (CH₂)ᵤ und zum anderen über Sauerstoff an das Kieselgel gebunden sind;
- m: den Wert einer Zahl von 1 bis 5000 hat;
- n: den Wert einer Zahl von 1 bis 5000 hat;
- x: den Wert einer Zahl von 1 bis 5000 hat;
- y: den Wert einer Zahl 1 bis 5000 hat;
- X: für eine direkte Bindung, Sauerstoff, Schwefel, -N(R¹)-, -C(=O)O-, -O(O=)C-, -N(R¹)(O=)C-, -C(=O)N(R¹)-, -O-CHR¹-O-, -OC(=O)N(R¹)-, - N(R¹)C(=O)O-, >C(=O) oder >C(=S) steht;
- R¹: für Wasserstoff oder C₁-C₄-Alkyl steht;
- R²: für Wasserstoff oder C₁-C₄-Alkyl steht;
- R³: für Wasserstoff oder C₁-C₄-Alkyl steht;
- R⁴: für Wasserstoff oder C₁-C₄-Alkyl steht;
- q: den Wert einer Zahl von 2 bis 5 hat,
- u: den Wert einer Zahl von 2 bis 5 hat, und
- K: ein Übergangsmetallkomplex ist,
wobei die mit m, n, x und y indizierten Monomereinheiten statistisch verteilt sind.

Das Molverhältnis von Styroleinheiten zu Acrylamideinheiten ist vorzugsweise wie oben für Formel II angegeben.

Die erfindungsgemäßen anorganischen Matrices mit daran gebundenem Acrylamid-Styrol-Copolymer, sprich anorganische Matrices, die Struktureinheiten der Formel (VII) umfassen: 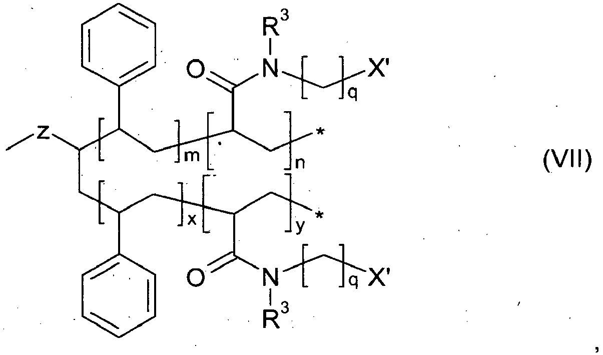 worin X' für eine Gruppe steht, die durch Umsetzung mit einem entsprechend modifizierten Übergangsmetallkomplex die Gruppe X bildet, z.B. OH, SH und NH₂, sind durch folgende Verfahrensschritte erhältlich:
i) Einführung einer Gruppe in die anorganische Matrix, die mit Styrol und/oder einem Acrylamid-Derivat der Formel (IX) copolymerisierbar ist, wobei diese Gruppe direkt oder über einen Spacer an die anorganische Matrix gebunden sein kann. Eine geeignete direkt an Matrix gebundene Gruppe ist die Vinylgruppe. Zu copolymeriserbaren über einen Spacer gebundenen Gruppen gehören eine Allylgruppe, Hydroxy-C₁-C₄-alkylacrylat, Hydroxy-C₁-C₄-alkylmethacrylat und insbesondere die Gruppe der Formel (VIII): 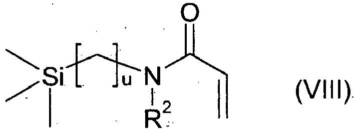 worin R² und u wie oben definiert sind. Die Einführung letzterer Gruppe kann beispielsweise durch Umsetzung der Matrix, insbesondere Kieselgel, mit (R₄O)₃Si-(CH₂)ᵤ-NHR² oder (R₄O)₃Si-(CH₂)ᵤ-OH in üblicher Weise erfolgen. Dabei bindet der Silanrest an 1, 2 oder 3 Atome der Matrix (Sauerstoffatome bei Kieselgel). Die auf diese Weise modifizierte Matrix wird dann beispielsweise mit Acrylsäurechlorid oder Methacrylsäurechlorid oder mit einem Acryl- oder Methacrylsäure-C₁-C₄-alkylester umgesetzt. Vorzugsweise jedoch wird (R₄O)₃Si-(CH₂)ᵤ-NHR² oder (R₄O)₃Si-(CH₂)ᵤ-OH mit Acrylsäurechlorid oder Methacrylsäurechlorid umgesetzt und das erhaltene Amid oder der erhaltene Ester wird dann an die Matrix gebunden.
ii) Copolymerisation der gemäß (i) erhaltenen modifizierten Matrix mit Styrol und einem Acrylamid-Derivat der Formel (IX): 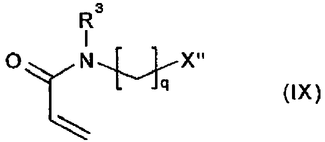 worin X" für gegebenenfalls geschütztes X' steht, z.B. für N-Acryloyl-N-methyl-propylphthalimid (ein Acrylamid-Derivat der Formel (IX), worin R³ Methyl ist, q = 3 ist und X" Phthalimidyl ist). Die relativen Anteile an den mit m, n, x, und y indizierten Monomereinheiten ergeben sich aus den zur Copolymerisation gewählten Einsatzmengen und können auch durch die Polymerisationsbedingungen eingestellt werden. Die Polymerisation erfolgt zweckmäßigerweise in einem inerten Lösungsmitten, wie Toluol unter Verwendung eines in dem Reaktionsgemisch löslichen Radikalinitiators, beispielsweise eine Azoverbindung, wie Azoisobutyronitril (AIBN);
iii) erforderlichenfalls Überführung der Gruppe X" in die Gruppe X', insbesondere durch Entschützen, beispielsweise durch Überführen der Phthalimidgruppe in eine Aminogruppe.

Der in der Regel ein oder mehrere Übergangsmetalle bzw. Übergangsmetallionen sowie einen oder mehrere Komplexliganden umfassende Übergangsmetallkomplex ist in der Regel über eine funktionelle Gruppe, die für eine unter den Anwendungsbedingungen stabile, in der Regel kovalente, Bindung sorgt, mit dem Träger obiger Ausführungen entsprechend entweder über die Ethylenglykol-Oligomere oder die Styrol-Acrylamid-Copolymere verbunden. Dabei stellt, wie oben ausgeführt, die Gruppierung X den Anknüpfungspunkt dar.

Erfindungsgemäß eignet sich der geträgerte Übergangsmetallkomplex als Olefinmetathese-Katalysator. Hierzu dienen Wolfram-, Molybdän- und Rutheniumalkyliden-Komplexe, von denen die

Molybdän- und insbesondere die Ruthenium-Komplexe erfindungsgemäß von besonderer Bedeutung sind.

Besagte Molybdän-Alkyliden-Komplexe besitzen beispielsweise eine Struktur der Formel (III): 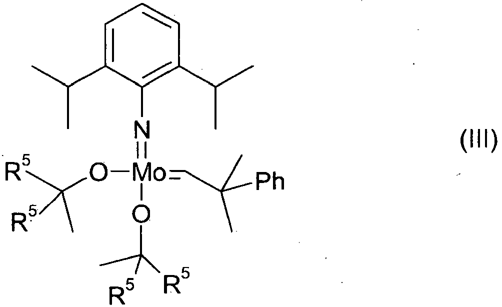 worin
- R⁵: für Methyl oder Trifluormethyl steht.

Derartige Molybdän-Alkyliden-Komplexe werden gemeinhin als Schrock-Katalysatoren bezeichnet.

Besagte Ruthenium-Alkyliden-Komplexe besitzen beispielsweise eine Struktur der Formel (IVa): 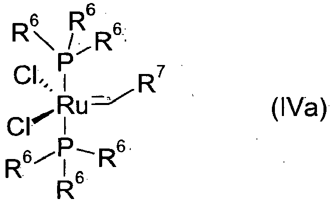 worin
- R⁶: für Phenyl oder Cyclohexyl steht, und
- R⁷: für Phenyl oder CH=CPh₂ steht.

Derartige Ruthenium-Alkyliden-Komplexe werden gemeinhin als Grubbs-Katalysatoren erster Generation bezeichnet.

Weitere Vertreter besagter Ruthenium-Alkyliden-Komplexe besitzen beispielsweise eine Struktur der Formel (IVb): 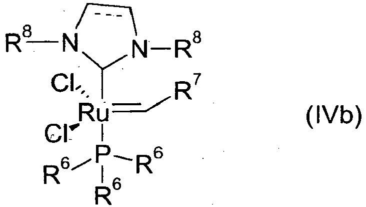 worin
- R⁶: Cyclohexyl ist;
- R⁷: Phenyl ist; und
- R⁸: 2,4,6-Trimethylphenyl (Mesityl) ist,
wobei "---" eine optionale Doppelbindung andeutet.

Derartige Ruthenium-Alkyliden-Komplexe werden gemeinhin als Grubbs-Katalysatoren zweiter Generation bezeichnet.

Weitere Vertreter besagter Ruthenium-Alkyliden-Komplexe besitzen beispielsweise eine Struktur der Formel (IVc): 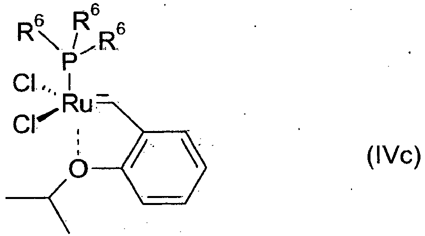 worin
- R⁶: Cyclohexyl ist.

Derartige Ruthenium-Alkyliden-Komplexe werden gemeinhin als Hoveyda-Katalysatoren erster Generation bezeichnet.

Weitere Vertreter besagter Ruthenium-Alkyliden-Komplexe besitzen die Struktur der Formel (IVd): 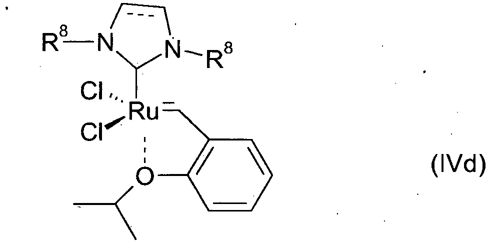 worin
- R⁸: für 2,4,6-Trimethylpheayl (Mesityl) steht,
wobei "---" eine optionale Doppelbindung andeutet.

Derartige Ruthenium-Alkyliden-Komplexe werden gemeinhin als Hoveyda-Katalysatoren zweiter Generation bezeichnet. Geträgerte Übergangsmetallkomplexe auf Basis von Hoveyda-Katalysatoren stellen eine besondere Ausführungsform der vorliegenden Erfindung dar.

In der erfindungsgemäßen geträgerten Form sind besagte Übergangsmetallkomplexe in der Regel modifiziert, wodurch eine Anbindung an den Träger, gewährleistet ist. Der Begriff "Übergangsmetallkomplex" in der vorliegend verwendeten Form bezieht sich also regelmäßig auf eine modifizierte Ausführung der an sich aus der homogenen Katalyse bekannten Übergangsmetallkomplexe, beispielsweise den oben beschriebenen Ruthenium-Alkyliden-Komplexen. So sind die Strukturen der Übergangsmetallkomplexe K in den vorliegende Formeln von den Strukturen der an sich aus der homogenen Katalyse bekannten Übergangsmetallkomplexe, beispielsweise den Strukturen der Formeln (IVa), (IVb), (IVc) oder (IVd); abgeleitet beziehungsweise beruhen auf diesen.

Zur Anbindung solcher Übergangsmetallkomplexe an die erfindungsgemäßen Träger, insbesondere den modifizierten Matrices der Formeln (VI) und (VII) sowie deren speziellenAusführungsformen, bieten sich vielfältige Möglichkeiten. In der Regel ist zurmindest einer der Übergangsmetallliganden mit einer funktionellen Gruppe versehen, welche die Anbindung gestattet. Dies soll am Beispiel der oben beschriebenen Ruthenium-Alkyliden-Komplexe beispielhaft erläutert werden. Prinzipiell können dazu folgende Vorgehensweisen unterschieden werden:
i) Austausch des Phosphinliganden;
ii) Austausch des Alkylidenliganden;
iii) Austausch des N-heterocyclischen Carbenliganden; oder
iv) Austausch eines Halogenliganden.

Ein Beispiel für die Vorgehensweise (i) ist der Austausch wenigstens eines Phosphinliganden, also insbesondere wenigstens einer Gruppe P(R⁶)₃ in den Komplexen der Formlen (IVa), (IVb) oder (IVc), gegen entsprechende Phosphine, in denen wenigstens ein Rest R⁶ derart modifiziert ist, dass hierüber eine Anbindung erfolgen kann. Auf diese Weise gelang es beispielsweise, Ruthenium-Alkyliden-Komplexe vom obigen Typ an eine mit 2 % Divinylbenzol quervernetzte Polystyrol-Matrix (S.T. Nguyen und R.H. Grubbs, J. Organomet. Chem. 1995, 497, 195-200) oder an Kieselgel (K. Mehlis, D. De Vos, P. Jakobs und F. Verpoort, J. Mol. Catal. A: Chem., 2001, 169, 47) zu binden.

Ein Beispiel für die Vorgehensweise (ii) ist der Austausch des AlkylidenLiganden, insbesondere der =CHR⁷-Gruppierung in den oben beschriebenen Ruthenium-Alkyliden-Komplexen der Formeln (IVa) und (IVb), gegen trägergebundenes Alkyliden. Auf diese Weise gelang es beispielsweise, Ruthenium-Alkyliden-Komplexe von obigem Typ an Poly(vinylstyrol-co-divinylbenzol) (M. Ahmed, A. G. M. Barrett, D.C. Braddock, S.M. Cramp, P.A. Procopiou, Tetrahedron Lett. 1999, 40, 8657-8662) zu binden.

Eine erfindungsgemäß bevorzugte Variante der Vorgehensweise (ii) bietet die Funktionalisierung des 2-Isopropoxybenzyliden-Liganden in den Ruthenium-Alkyliden-Komplexen vom Typ der Formeln (IVc) oder (IVd). So lassen sich insbesondere in 5-Position geeignete Substituenten einführen. Hierzu gehören Carboxyalkylgruppen, beispielsweise -CH₂-CH₂-COOH (vgl. S. B. Garber, J.S. Kingsbury, B.L. Gray, A. H. Hoveyda, J. Am. Chem. Soc. 2000, 122, 8168-8179; J. S. Kingsbury, S. B. Garber, J. M. Giftos, B. L. Gray, M.M. Okamoto, R.A. Farrer, J.T. Fourkas, A.H. Hoveyda, Angew. Chem. 2001, 113, 4381-4386; Angew. Chem, Int. Ed. 2001, 40, 4251-4256; J. S. Kingsbury, A.H. Hoveyda, J. Am Chem. Soc. 2005, 127, 4510-4517; S.J. Connon, S. Blechert, Bioorg. Med. Chem. Lett. 2002, 12, 1873-1876), Carboxyalkenylgruppen, beispielsweise -CH=CH-COOH (F. Koç, F. Michalek, L. Rumi, W. Bannwarth, R. Haag, Synthesis 2005, 3362-3372), eine Hydroxygruppe (Q. Yao, Angew. Chem. 2000,112, 4060-6042; Angew. Chem. Int. Ed. 2000, 39, 3896-3898; S. RandI, N. Buschmann, S.J. Connon, S. Blechert, Synlett 2001, 1547-1550), Hydroxyalkylgruppen, beispielsweise Hydroxymethyl (Q. Yao, A.R. Motta, Tetrahedron Lett. 2004, 45, 2447-2451), oder Halogenatome, beispielsweise Brom (K. Grela, M. Tryznowski, M. Bieniek, Tetrahedron Lett. 2002, 43, 9055-9059).

Eine weitere Möglichkeit besteht darin, einen ähnlichen Substituenten in einer anderen Position als der 5-Position des 2-Isopropoxybenzyliden-Liganden einzuführen, beispielsweise eine Hydroxygruppe in 3-Position (C. Fischer, S. Blechert, Adv. Synth. Catal. 2005, 347, 1329-1332).

Alternativ zur Einführung eines Substituenten am Phenylring des Benzyüdens bietet sich die Modifizierung des Isopropoxy-Substituenten in 2-Position des Benzyliden-Liganden in den Ruthenium-Alkyliden-Komplexen vom Typ der Formeln (IVc) oder (IVd) an. Beispielsweise lässt sich die Isopropoxygruppe durch eine 1-Carboxyhexan-2-oxy-Gruppe ersetzen (J. Dowden, J. Savovic, Chem. Commun. 2001, 37-38).

Ein Beispiel für die Vorgehensweise (iii) ist die Einführung eines Substituenten in 4-Position des 1,3-disubstituierten Imidazol-2-yliden- oder 4,5-Dihydroimidazol-2-yliden-Liganden, oder ein Austausch eines oder beider Substituenten R⁸ in den Ruthenium-Alkyliden-Komplexen der Formeln (IVb) und (IVd) dergestalt, dass hierüber eine Anbindung möglich ist. Beispielsweise lassen sich Hydroxyalkylgruppen, beispielsweise Hydroxymethyl, in 4-Position des Liganden einführen (S. Randl, N. Buschmann, S.J. Connon. S, Blechert, Synletf 2001, 1547-1550; S. C. Schürer, S. Gessler, N. Buschmann, S. Blechert, Angew. Chem. 2000, 12, 4062-4065; Angew. Chem. Int Ed. 2000, 39, 3898-3901) oder eine Hydroxyalkylgruppe, beispielsweise Hydroxyhexyl, an einen Stickstoff des Liganden binden (S. Prühs, C.W. Lehmann, A. Fürstner, Organometallics 2004, 23, 280-287).

Ein Beispiel für die Vorgehensweise (iv) ist der Austausch eines Halogens der oben beschriebenen Ruthenium-Alkyliden-Komplexe durch geeignete, entsprechend funktionalisierte Liganden, wie Perfluorglutarsäure (J. O. Krause, S. Lubbad, O. Nuyken und M. R. Buchmeiser, Adv. Synth. Catal., 2003, 345, 996).

Derart modifizierte Übergangsmetallkomplexe können an den Träger (d.h. an die Gruppe X' unter Bildung der Gruppe X, direkt oder unter Zwischenschaltung eines Spacers, gebunden sein.

Obigen Ausführungen entsprechend ergeben sich somit vielfältige Anbindungsmöglichkeiten von Übergangsmetallkomplexen an die erfindungsgemäßen Träger.

Erfindungsgemäß insbesondere bevorzugt ist es, wenn in den geträgerten Übergangsmetallkomplexen der Formeln (la) K für eine Gruppe der Formel (V) steht: 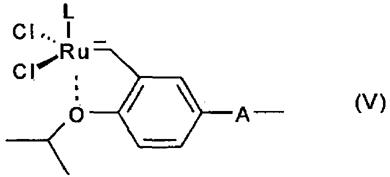 worin
L für P(R⁶)₃, oder 1,3-substituiertes 4,5-Dihydroimidazol-2-yliden steht, wobei R⁶ wie oben definiert ist; und
A für einen an X gebundenen 2-wertigen Rest steht, der vorzugsweise ausgewählt ist unter C₁-C₁₀-Alkylen, C₂-C₁₀-Alkenylen, wobei die Alkenyl- und Alkenylen-Reste durch 1, 2 oder 3 Heteroatome unterbrochen sein können, die vorzugsweise ausgewählt sind unter Sauerstoff, Stickstoff und Schwefel.

Gemäß einer besonders bevorzugten Ausführungsform steht A für Methylen, Ethylen, Propylen oder Ethenylen.

Die erfindungsgemäßen geträgerten Übergangsmetallkomplexe finden Anwendung in der Olefinmetathese.

Die Verwendung der oben beschriebenen geträgerten Ruthenium-Alkyliden-Komplexe in der Olefinmetathese stellt eine besondere Verwendung im Sinne der Erfindung dar.

Das erfindungsgemäße Verfahren zur übergangsmetallkatalysierten Umsetzung von Edukt(en) zu Produkt(en) ist die Olefinmetathese. Hierbei handelt es sich erfindungsgemäß um eine übergangsmetallkatalysierte Reaktion, bei der zwischen zwei substituierten Alkenen formal die Alkylidengruppen ausgetauscht werden. Es handelt sich somit um ein katalytisches Verfahren zur Spaltung und Knüpfung von C-C-Doppelbindungen.

Im Bereich der Olefinmetathese lassen sich je nach umzusetzenden Edukten und den zu erwartenden Produkten verschiedene Ausführüngsformen unterscheiden. Zu den wichtigsten Vertretern zählen die Ringöffnungsmetathese-Polymerisation (kurz ROMP für englisch: Ring-Opening Metathesis Polymerisation), die acyclische Dien-Metathese (kurz ADMET für englisch: Acyclic Dien Metathesis), die Kreuzmetathese (kurz CM für englisch: Cross Metathesis), die Ringöffnungsmetathese (kurz ROM für englisch: Ring-Opening Metathesis), und die Ringschlussmetathese (kurz RCM für englisch: Ring-Closing Metathesis). Ferner sind noch die 1-Alkinpolymerisation, die Enin-Metathese, die Ringöffnungskreuzmetathese und die Tandemmetathese, beispielsweise die Tandem-Ringöffnungs-Ringschluss-Metathese und die kombinierte Ringöffnung-Ringschluss-Kreuzmetathese, von Bedeutung.

Gemäß einer besonderen Ausführungsform betrifft die vorliegende Erfindung ein Verfahren zur übergangsmetallkatalysierten Ringschlussmetathese. Hierbei handelt es sich insbesondere um eine intramolekulare Umsetzung von α,ω-Diolefinen zu entsprechenden cyclischen Produkten. α,ω-Diolefine sind Verbindung, die zwei terminale Doppelbindung umfassen.

Mit Hilfe der erfindungsgemäßen geträgerten Übergangsmetallkomplexe ist es möglich, einen Großteil des (oder der) eingetragenen Edukts (oder Edukte) in kurzer Zeit insbesondere in überkritischem Kohlendioxid umzusetzen. Die Optimierung der Reaktionsbedingungen, beispielsweise die Reaktionstemperatur, der Kohlendioxiddruck, die Reaktionsdauer sowie die einzusetzenden Mengen an Edukt und geträgertem Übergangsmetallkomplex, erfolgt durch den Fachmann. Beispielsweise lassen sich (α,ω-Diolefine im Sinne einer Ringschlussmetathese zu entsprechenden cyclischen Produkten bei einer Temperatur im Bereich von 20 bis 60 °C, insbesondere 30 bis 50 °C, beispielsweise bei etwa 40 °C, einem Kohlendioxiddruck im Bereich von 80 bis 160 bar, beispielsweise bei etwa 150 bar, einer Konzentration an geträgertem Übergangsmetallkömplex im Bereich von 1 bis 5 mol-%, vorzugsweise 2 bis 3 mol-%, beispielsweise etwa 2,5 mol-% (Ru bezogen auf die Menge an Edukt), in wenigen Stunden, beispielsweise in 10 bis 24 Stunden, zu von mehr-als 80 % umsetzen.

Geeignete Vorrichtungen zur Durchführung von Umsetzungen in überkritischem Kohlendioxid sind dem Fachmann genauso bekannt wie die Einstellung des überkritischen Zustands.

Die aus der Umsetzung resultierenden Wertprodukte können in an sich bekannter Weise gewonnen werden.

In obigen Formeln steht C₁-C₄-Alkyl für Methyl, Methyl, n-Propyl, i-Propyl, n-Butyl, sec-Butyl, i-Butyl oder t-Butyl. Bevorzugt sind Ethyl und insbesondere Methyl.

Die folgenden Beispiele veranschaulichen die Erfindung, ohne sie jedoch zu beschränken.

### Beispiele

### Beispiel 1: Herstellung des geträgerten Übergangsmetallkomplexes

### 1: Herstellung von (E)-3-(4-Isopropoxy-3-vinylphenyl)acrylsäure

(E)-3-(4-Isopropoxy-3-vinylphenyl)acrylsäure wurde hergestellt, indem man 5-Brom-2-hydroxybenzaldehyd in DMF in Gegenwart von K₂CO₃ und Cs₂CO₃ mit 2-Iodpropan zum 5-Brom-2-isopropoxybenzaldehyd, diesen dann mit Ethylacrylat in wasserfreiem DMF (Dimethylformamid) in Gegenwart von Pd(OAc)₂ und P(o-Tol)₃ sowie Triethylamin zum (E)-3-(3-Formyl-4-isopropoxyphenyl)ethylacrylat, dieses mit Methyltriphenylphosphoniumbromid und BuLi in wasserfreiem THF (Tetrahydrofuran) zum (E)-3-(4-Isopropoxy-3-vinylphenyl)ethylacrylat, und dieses schließlich in 1,4-Dioxan mit einer wässrigen KOH-Lösung zur (E)-3-(4-Isopropoxy-3-vinylphenyl)acrylsäure umsetzte. Diese Reaktionssequenz ist in F. Koç, F. Michalek, L. Rumi, W. Bannwarth, R. Haag, Synthesis 2005, 3362-3372 genauer beschrieben.

### 2: Kopplung der (E)-3-(4-Isopropoxy-3-vinylphenyl)acrylsäure an Aminogruppen vierschiedener Träger

Man koppelte die (E)-3-(4-Isopropoxy-3-vinylphenyl)acrylsäure an gegebenenfalls entschützte Aminogruppen des betreffenden Trägers, indem man die Acrylsäure zusammen mit HOBt in DMF löste, DCC (Dicyclohexylcarbodiimid) und Huenig's Base zu dieser Lösung gab und das resultierende Kopplungsgemisch zu dem in frische DMF suspendierten Träger gab. Sobald keine freien Aminogruppen mehr feststellbar waren, setzte man die feste Phase mit Triethylamin und DMAP in Dichlormethan sowie einer Acetanhydridlösung um.

Diese Reaktion ist in F. Koç, F. Michalek, L. Rumi, W. Bannwarth, R. Haag, Synthesis 2005, 3362-3372 genauer beschrieben.

Auf diese Weise koppelte man (E)-3-(4-Isopropoxy-3-vinylphenyl)acrylsäure an folgende Träger:

### a) HypoGel® 400

HypoGel® 400 ist der Handelsname für ein von Rapp Polymere, Tübingen (Deutschland) vertriebenes hydrophiles Harz auf Basis einer Polystyrol-Matrix mit geringem Vernetzungsgrad (1 % Divinylbenzol), welche Struktureinheiten der Formel (VIa) umfasst: 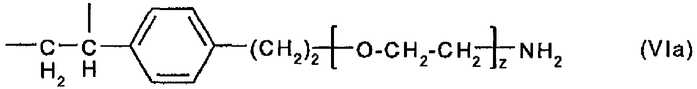 worin z den Wert 10 hat.

### b) Hybrid-Kieselgel

Hybrid-Kieselgel steht für einen Träger auf Kieselgel-Basis mit einer Beschichtung aus Acrylamid-Styrol-Copolymer. Die Acrylamid-Einheiten sind alkyliert, und zwar jeweils mit einer Methylgruppe und einer Propylengruppe, die entweder eine freie Aminogruppe aufweist oder über Alkoxysilangruppen an das Kieselgel gebunden ist. Der Träger weist demnach eine Kieselgel-Matrix auf, die Struktureinheiten der folgenden Formel (VII) umfasst: 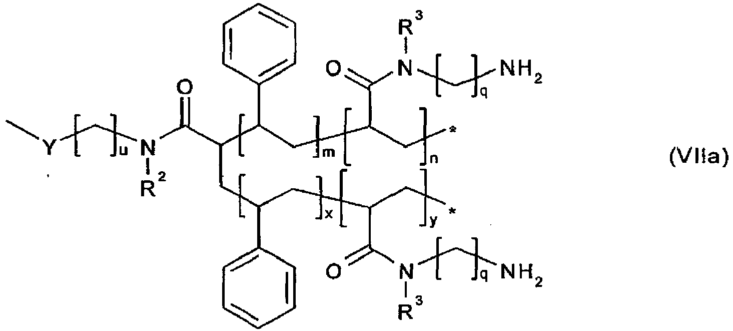 worin
Y für Si, Si(OR⁴) oder Si(OR⁴)₂ steht, wobei die freien Valenzen des Siliziums zum einen an das Alkylen (CH₂)ᵤ und zum anderen über Sauerstoff an das Kieselgel gebunden sind;
m für einen Wert von 1 bis 5000 steht;
n für einen Wert von 1 bis 5000 steht;
x für einen Wert von 1 bis 5000 steht;
y für einen Wert von 1 bis 5000 steht; und
R² und R³ Methyl sind;
R⁴ Methyl ist; und
u und q jeweils den Wert 3 haben.

Hybrid-Kieselgel steht daher für einen Träger, der eine vergleichsweise starre Kernstruktur aufweist, die mit einer ultradünnen Schicht (im Nanometerbereich) eines Acrylamid-Styrol-Copolymers überzogen ist.

Die Herstellung des Hybrid-Kieselgels wird nachfolgend näher erläutert:

Die zur Anwendung kommenden Träger auf Siliciumdioxidbasis wurden unter trockenem Stickstoff in Toluol suspendiert. Anschließend wurden die Silanmonomereinheit und Triethylamin, gelöst in Toluol, zugegeben, bis zum beginnenden Rückfluss (120 °C) erhitzt und 3h mit einer Schüttelvorrichtung in Bewegung gehalten. Die durchgeführten Versuche sind in der nachfolgenden Tabelle 1 zusammengestellt:

**Tabelle 1: Rezepturen**

| **modifizierter Träger¹⁾** | **mmol Silan** | **Menge Träger (g)** | **Volumen Toluol (mL)** | **Menge Tri ethylamin (mmol)** |
|---|---|---|---|---|
| LC700-ME | 8.4 | 6 | 200 | 54 |
| GB80-ME | 4.2 | 10 | 125 | 36 |
| GB80-AA | 4.2 | 10 | 80 | 28 |
| GB250-AA | 2.5 | 5 | 60 | 21 |

| | | | | |
|---|---|---|---|---|
| ¹⁾ LC 700 (Lichrosphere) ist ein handelsübliches Kieselgel, GB 80 und 250 sind Glasbeads unterschiedlichen Durchmessers. Der Zusatz ME bedeutet, dass der Träger unter Verwendung von (MeO₃)Si(CH₂)₃OH und Methacrylsäurechlorid modifiziert wurde (beim Zusatz AE wurde statt Methacrylsäurechlorid Acrylsäurechlorid verwendet). Der Zusatz AA bedeutet, dass der Träger unter Verwendung von (MeO₃)Si(CH₂)₃NHCH₃ und Acrylsäurechlorid modifiziert wurde. | | | | |

Die modifizierten Träger auf Kieselgelbasis wurden bei 12500 Upm zentrifugiert (die modifizierten Glasbeads haben sich rasch genug abgesetzt, so dass kein Zentrifugieren erforderlich war) und mit Toluol, Ethanol, Ethanol/Wasser (1/1, V/V, mit HCl angesäuert), Ethanol/Wasser (1/1, V/V), Ethanol und Diethylether gewaschen, um überschüssiges Triethylamin zu entfernen. Die erhaltenen farblosen Produkte wurden 18h bei 0,01 mbar getrocknet.

### Copolymerisation zu matrixgebundenenm Poly(styrol-co-N-acryloyl-N-methyl-propylphthalimid, PS-AC3pht

Die modifizierten Träger wurden in einem Schlenk-Rohr mit Toluol und Styrol (Hauptmonomer) in den in Tabelle 2 angegebenen Mengen versetzt. Anschließend wurden N-Acryloyl-N-methyl-propylphthalimid (funktionalisiertes Monomer) und AIBN in der flüssigen Phase in Lösung gebracht. Die Lösung wurde im Vakuum durch 5 Gefrier-Tau-Zyklen entgast und das Gemisch wurde auf 60,0 °C thermostatisiert. Nach einiger Zeit wurde die Polymerisation durch Lufteinlaß und Abkühlen gestoppt. Die gewünschten Produkte wurden durch Zentrifugieren oder Absetzen lassen abgetrennt. Die Polymerisationsbedingungen sind in der Tabelle 2 angegeben.

**Tabelle 2: Copolymerisationen**

| **Modifizierter Träger** | **Menge Träger (mg)** | **Menge fM (mg)** | **Konz. fM (mol%)** | **Vol. M (mL)** | **Vol. Lösgs mittel (mL)** | **Menge I (mg)** | **Konz. I (mmol·L⁻¹)** | **t (h)** |
|---|---|---|---|---|---|---|---|---|
| LC700-ME | 240 | 946 | 5 | 8.0 | 16 | 36 | 9 | 24 |
| LC700-ME | 5000 | 1,320 | 10 | 5.0 | 10^{#} | 22 | 9 | 30 |
| GB80-ME | 2000 | 1,426 | 5 | 12.0 | 24 | 18 | 3 | 24 |
| GB80-ME | 2000 | 2,836 | 10 | 10.8 | 24 | 17 | 3 | 25 |
| GB80-ME | 2000 | 5,673 | 20 | 9.6 | 24 | 17 | 3 | 25 |
| GB80-AA | 13500 | 3,160 | 10 | 12.0 | 100 | 80 | 5 | 30 |
| GB250-AA | 5000 | 2,370 | 20 | 4.0 | 8# | 18 | 9 | 26 |

In der Tabelle bedeuten: fM = funktionalisiertes Monomer; M = Hauptmonomer; I = Initiator

### Matrixgebündenes Poly(styrol-co-N-acryloyl-N-methyl-propylamin), PS-AC3Amin

Die Copolymerisate der vorangehenden Stufe wurden mit THF überschichtet und mit Hydrazin-hydrat (bis zum 50fachen Überschuss) versetzt. Das Gemisch wurde 18h bei 60 °C und bei 180 Upm geschüttelt und anschließend filtriert, wobei die verbleibenden Feststoffe mit Toluol, Dichlormethan und erneut Toluol gewaschen wurden. Anschließend erfolgte Gefriertrocknen aus Benzol.

Zur Quantifizierung der Anzahl an Aminogruppen wurde ein DMT-Farbstoff mit Disulfidlinker an die Aminofunktionen gekoppelt. Bestimmung des Schwefelgehaltes und Abspaltung des Farbstoffes für die UV-Bestimmung ergab die Menge an zugänglichen Aminogruppen, siehe die nachfolgende Tabelle 3.

Im Falle der Glasbeads wurde die Phthalimidabspaltung mit Methylamin durchgeführt. Eine Suspension von 1,9 g des Phthalimids und 20 ml einer 2M Methylaminlösung in THF wurde auf 60 °C erhitzt und 18h bei 160 Upm geschüttelt. Die Glasbeads setzten sich beim Abkühlen ab, sie wurden 6x mit je 40 mL THF und 2x mit je 40 mL Diethylether gewaschen und 8h im Vakuum getrocknet.

**Tabelle 3: Analysenergebnisse**

| **Produkt** | **C* (%)** | **H* (%)** | **N* (%)** | **m'_{poly}^{#} (g)** | **δ(NH₂)° (µmol/g )** |
|---|---|---|---|---|---|
| LC700-ME-PS-AC₃amine(10%) | 25.60 | 3.43 | 0.53 | 0.350 | 20 |
| GB80-ME-PS-AC₃amine(5%) | 11.00 | 0.86 | 0.22 | 0.115 | - |
| GB80-ME-PS-AC₃amine(10%) | 11.17 | 0.86 | 0.33 | 0.120 | 90 |
| GB80-ME-PS-AC₃amine(20%) | 11.84 | 1.14 | 0.66 | 0.139 | 90 |
| GB80-AA-PS-AC₃amine(10%) | 7.83 | 1.19 | 0.60 | 0.081 | 60-80 |
| GB250-AA- PS-AC₃amine(20%) | 19.46 | 2.51 | 1.62 | 0.242 | 150 |

| | | | | | |
|---|---|---|---|---|---|
| * erhalten durch Elementaranalyse # pro g SiO₂, bestimmt aus der Elementaranalyse ° Messungen der gekoppelten DMT-Disulfid-Einheiten | | | | | |

### 4: Beladung mit Ruthenium

Man suspendierte den betreffenden Träger mit daran gebundener (E)-3-(4-Isopropoxy-vinylphenyl)acrylsäure in wasserfreiem Dichlormethan unter Argonatmosphäre und gab entweder (PCy₃)₂Cl₂Ru=CHPh oder (4,5-H₂IMES)(PCy₃)Cl₂Ru=CHPh (Cy = Cyclohexyl; IMES = 1,3-Bis(2,5,6-trimethylphenyl)imidazol-2-yliden) sowie CuCl dazu.

Diese Umsetzung ist in F. Koç, F. Michalek, L. Rumi, W. Bannwarth, R. Haag, *Synthesis* 2005, 3362-3372 genauer beschrieben.

Zur Entfernung unlöslicher Cu-Phosphinreste ging man folgendermaßen vor:

Im Falle von Hybrid-Siligagel-geträgerten Übergangsmetallkomplexen filtrierte man das Reaktionsgemisch und wusch das Filtrat, bis es farblos war. Anschließend gab man Neocuproin dazu, um Cu-Ionen spezifisch zu entfernen, und man betätigte ihre Abwesenheit mittels XPS-Messungen.

Im Falle von HypoGel® 400-geträgerten Übergangsmetallkomplexen ging man so vor wie in S.J. Connon, S. Blechert, Bioorg. Med. Chem. Lett. 2002, 12, 1873-1876 beschrieben.

Anschließend bestimmte man die Ru-Beladung sämtlicher geträgerter Übergangsmetallkomplexe mittels ICP-MS (im Falle von Hybrid-Kieselgel geträgerten Übergangsmetallkomplexen) oder mittels AAS (im Falle von HypoGel® 400-geträgerten Übergangsmetallkomplexen). Die Ergebnisse sind in folgender Tabelle 4 zusammengestellt:

**Tabelle 4**

| Träger | Ligand | Beladung [µmol/g] |
|---|---|---|
| Hybrid-Kieselgel | PCy₃ | 32 |
| | H₂IMES | 56 |
| HypoGel® 400 | PCy₃ | 67 |
| | H₂IMES | 217 |

### Beispiel 2: Katalyse

Die Reaktionen wurden in einem Stahlautoklaven derNWA GmbH, Lörrach (Deutschland) (variables Volumen von 29-61 ml) durchgeführt. Der Autoklav war mit einem Saphir-Fenster und einem Innenrührer bestückt. Man verwendete ein Druckmodul mit einem Maximalausstoß von 600 bar, um Kohlendioxid einzuleiten.

Die allgemeine Vorgehensweise der katalytischen Umsetzung unter überkritischen Bedingungen war wie folgt: Man gab den geträgerten Übergangsmetallkomplex (2,5 mol-%) in ein speziell gestaltetes kleines Glasgefäß im Autoklaven. Der Reaktor wurde bei 40 °C vorsichtig mit CO₂ bis auf 100 bar unter Druck gesetzt. Dann gab man das Substrat über eine mit dem Autoklaven verbundene Schleife zu und erhöhte den Druck auf 140 bar. Nach 24 Stunden lüftete man den Reaktor bei 40 °C. Man gewann die organischen Verbindungen in einem mit Dichlormethan und Ethylvinylether befüllten Kolben. Der Umsatz wurde dann mittels ¹H-NMR bestimmt. Sämtliche Produkte wurden mittels ¹H-NMR und Massenspektrometrie analysiert und mit den Literaturangaben verglichen.

Die katalytischen Eigenschaften der geträgerten Übergangsmetallkomplexe wurden anhand der Umsetzung von N,N-Diallyltosylamid zu N-Tosylpyrrolin mittels Ringschlussmetathese beurteilt.

In der folgenden Tabelle 5 sind die Umsätze für jeden der verwendeten geträgerten Übergangsmetallkomplexe zusammengestellt:

**Tabelle 5**

| Träger | Ligand | Umsatz (Zyklus 1,2,3,4,5) [%] |
|---|---|---|
| Hybrid-Kieselgel | PCy₃ | 83, 75, 66, 66, 56 |
| | H₂IMES | 93, 92, 77, 4 57 |
| HypoGel® 400 | PCy₃ | 95, 95, 90, 89, 84 |
| | H₂IMES | 92, 95, 85, 34, 12 |

Zum Vergleich wurde die gleiche Umsetzung mit den entsprechenden ungeträgerten Übergangsmetallkomplexen (PCy₃)₂Cl₂Ru=CHPh und (H₂IMES)(PCy₃)Cl₂Ru=CHPh durchgeführt. Die erzielten Umsätze sind in der folgenden Tabelle 6 zusammengestellt:

**Tabelle 6**

| Übergangsmetallkomplex | Umsatz (Zyklus 1,2,3,4,5) [%] |
|---|---|
| (PCy₃)₂Cl₂Ru | >98, >98, >98, >98, >98 |
| (H₂IMES)(PCy₃)Cl₂Ru | >98, >98, >98, >98, 90 |

Die Vergleichsübergangsmetallkomplexe (PCy₃)₂Cl₂Ru bzw. (H₂IMES)(PCy₃)Cl₂Ru ergaben jeweils eine quantitative Umsetzung des Edukts. Es waren 5 aufeinanderfolgende Durchgänge ohne Aktivitätsverlust möglich.

Obwohl nicht vollständig, zeigten die geträgerten Übergangsmetallkomplexen ähnlich gute Umsetzungen.

Weitere Untersuchungen mit Hybrid-Kieselgel-geträgertem (H₂IMES)(PCy₃)Cl₂Ru = CHPh zeigten, dass die Umsetzung im Bereich von 80 und 160 bar Kohlendioxid druckunabhängig war:

Zeitabhängige Untersuchungen zeigten ferner, dass die Umsetzung bei Verwendung der Vergleichsübergangsmetallkomplexe bereits nach einer Stunde vollständig war, wohingegen bei Verwendung des Hybrid-Kieselgel-geträgerten Übergangsmetallkomplexes zu diesem Zeitpunkt lediglich 50 % umgesetzt waren (eine 90 %ige Umsetzung wurde nach etwa 6 Stunden erreicht).

Überraschenderweise betrug der mittels ICP-AES bestimmte Ru-Gehalt des Produkts (N-Tosylpyrrolin) bei Verwendung der beiden Hybrid-Kieselgel-geträgerten Übergangsmetallkomplexe lediglich 18 bzw. 21 ppm, wohingegen das Produkt bei Verwendung der Vergleichsübergangsmetallkomplexe mit 100 ppm verunreinigt war. Ein Gehalt von lediglich etwa 20 ppm Ruthenium bei einem Umsatz von über 90 % ist bemerkenswert.

In der folgenden Tabelle 7 finden sich Angaben zu weiteren Edukten und dazugehörigen Ringschlussmetathese-Produkten sowie dem bei Verwendung des Hybrid-Kieselgel-geträgerten (H₂IMES)(PCy₃)Cl₂Ru=CHPh erzielbaren Umsatz.

**Tabelle 7**

| Edukt | Produkt | Umsatz [%] |
|---|---|---|
| 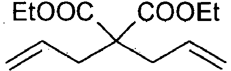 | 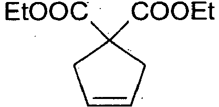 | 74 |
| 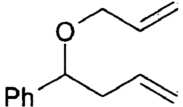 | 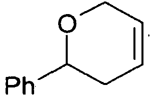 | 90 |
| 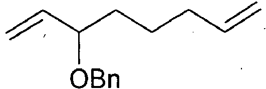 | 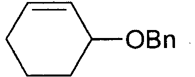 | > 98 |
| 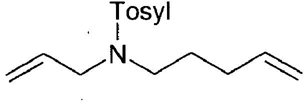 | 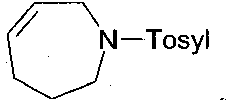 | > 98 |
| 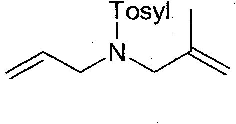 | 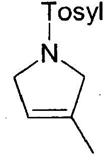 | 92 |
| 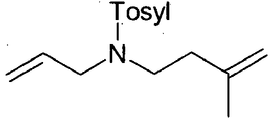 | 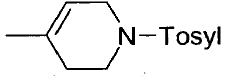 | 93 |

## Patentansprüche

1. Olefinmetathese-Katalysator in Form eines geträgerten Übergangsmetallkomplexes auf Basis einer Polystyrol-Matrix, die Struktureinheiten der Formel (la): 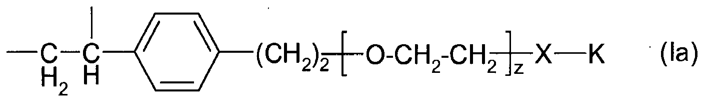 umfasst, worin
z den Wert einer Zahl von 3 bis 20 hat;
X für eine direkte Bindung, Sauerstoff, Schwefel, -N(R¹)-, -C(=O)O-, -O(O=)C-, -N(R¹)(O=)C-, -C(=O)N(R¹)-, -O-CHR¹-O-, -OC(=O)N(R¹)-, - N(R¹)C(=O)O-, >C(=O) oder >C(=S) steht;
R¹ für Wasserstoff oder C₁-C₄-Alkyl steht; und
K ein Wolfram-, Molybdän- oder Rutheniumalkylidenkomplex ist.

2. Olefinmetathese-Katalysator in Form eines geträgerten Übergangsmetallkomplexes nach Anspruch 1, **dadurch gekennzeichnet, dass** z = 10 ist.

3. Olefinmetathese-Katalysator in Form eines geträgerten Übergangsmetallkomplexes auf Basis einer Kieselgel-Matrix, die Struktureinheiten der Formel (II): 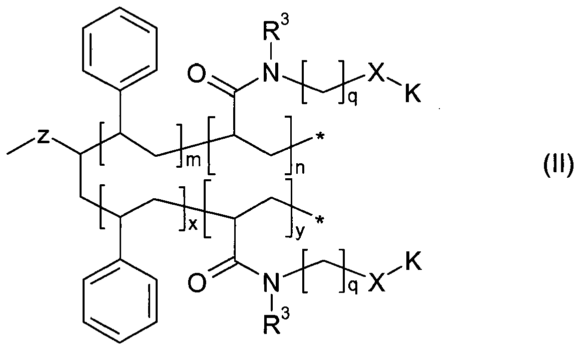 umfasst, worin
Z für eine Bindung oder einen Spacer steht;;
m den Wert einer Zahl von 1 bis 5000 hat;
n den Wert einer Zahl von 1 bis 5000 hat;
x den Wert einer Zahl von 1 bis 5000 hat;
y den Wert einer Zahl von 1 bis 5000 hat;
X für eine direkte Bindung, Sauerstoff, Schwefel, -N(R¹)-, -C(=O)O-, -O(O=)C-, -N(R¹)(O=)C-, -C(=O)N(R¹)-, -O-CHR¹-O-, -OC(=O)N(R¹)-, - N(R¹)C(=O)O-, >C(=O) oder >C(=S) steht;
R¹ für Wasserstoff oder C₁-C₄-Alkyl steht;
R³ für Wasserstoff oder C₁-C₄-Alkyl steht;
q den Wert einer Zahl von 2 bis 5 hat,
K ein Wolfram-, Molybdän- oder Rutheniumalkylidenkomplex ist.

4. Olefinmetathese-Katalysator in Form eines geträgerten Übergangsmetallkomplexes nach Anspruch 3, **dadurch gekennzeichnet, dass** q = 3 ist.

5. Olefinmetathese-Katalysator in Form eines geträgerten Übergangsmetallkomplexes nach einem der Ansprüche 3 bis 4, **dadurch gekennzeichnet, dass** R³ Methyl ist.

6. Olefinmetathese-Katalysator in Form eines geträgerten Übergangsmetallkomplexes nach einem der Ansprüche 1 bis 5, **dadurch gekennzeichnet, dass** X eine Amidbindung, insbesondere -N(H)(O=)C-, ist.

7. Olefinmetathese-Katalysator in Form eines geträgerten Übergangsmetallkomplexes nach einem der Ansprüche 1 bis 6, **dadurch gekennzeichnet, dass** der Übergangsmetallkomplex ein Molybdän- oder Ruthenium-Alkyliden-Komplex ist.

8. Olefinmetathese-Katalysator in Form eines geträgerten Übergangsmetallkomplexes nach Anspruch 7, **dadurch gekennzeichnet, dass** der Molybdän- oder Ruthenium-Alkyliden-Komplex sich von einer der folgenden Strukturen ableitet: einer Struktur der Formel (III): 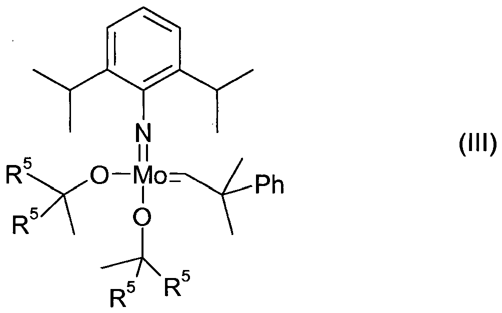 worin
R⁵ für Methyl oder Trifluormethyl steht, oder
einer Struktur der Formel (IVa): 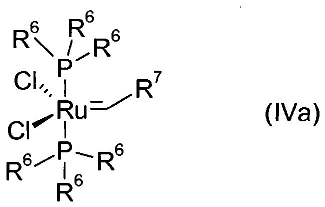 worin
R⁶ für Phenyl oder Cyclohexyl steht, und
R⁷ für Phenyl oder CH=CPh₂ steht, oder
einer Struktur der Formel (IVb): 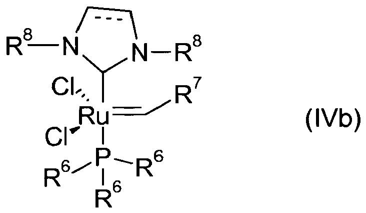 worin
R⁶ Cyclohexyl ist;
R⁷ Phenyl ist; und
R⁸ 2,4,6-Trimethylphenyl (Mesityl) ist,
wobei "---" eine optionale Doppelbindung andeutet, oder einer Struktur der Formel (IVc): 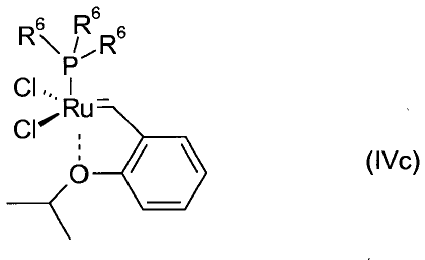 worin
R⁶ Cyclohexyl ist, oder
einer Struktur der Formel (IVd): 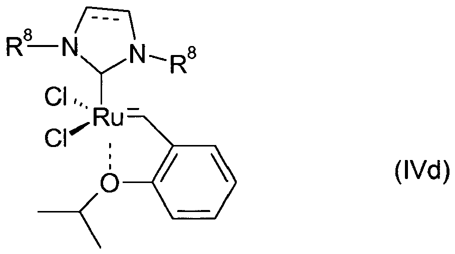 worin
R⁸ 2,4,6-Trimethylphenyl (Mesityl) ist,
wobei "---" eine optionale Doppelbindung andeutet.

9. Olefinmetathese-Katalysator in Form eines geträgerten Übergangsmetallkomplexes nach einem der Ansprüche 1 bis 8, **dadurch gekennzeichnet, dass** K für eine Gruppe der Formel (V) steht: 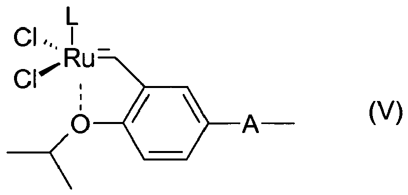 worin
L für P(R⁶)₃ oder 1,3-substituiertes 4,5-Dihydroimidazol-2-yliden steht, wobei R⁶ für Cyclohexyl steht; und
A für an X gebundenes C₁-C₁₀-Alkylen oder C₂-C₁₀-Alkenylen steht.

10. Verwendung eines Olefinmetathese-Katalysators in Form eines geträgerten Übergangsmetallkomplexes nach einem der Ansprüche 1 bis 9 in der Olefinmetathese.

11. Verfahren zur übergangsmetallkatalysierten Olefinmetathese in überkritischem Kohlendioxid, **dadurch gekennzeichnet, dass** der Katalysator ein Olefinmetathese-Katalysator in Form eines geträgerten Übergangsmetallkomplexes nach einem der Ansprüche 1 bis 9 ist.

12. Verfahren nach Anspruch 11, **dadurch gekennzeichnet, dass** die Olefinmetathese eine Ringschlussmetathese ist.

13. Verfahren nach Anspruch 11 oder 12, **dadurch gekennzeichnet, dass** das Verfahren kontinuierlich geführt wird.

## Claims

1. Olefin metathesis catalyst in the form of a supported transition metal complex based on a polystyrene matrix comprising structural units of the formula (Ia) : 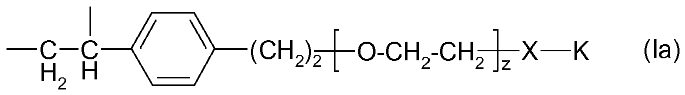 where
z is from 3 to 20;
X is a direct bond, oxygen, sulfur, -N(R¹)-, -C(=O)O-, -O(O=)C-, -N(R¹) (O=)C-, -C(=O)N(R¹)-, -O-CHR¹-O-, -OC(=O)N(R¹)-, - N(R¹)C(=O)O-, >C(=O) or >C(=S);
R¹ is hydrogen or C₁-C₄-alkyl; and
K is a tungsten-, molybdenum- or ruthenium-alkylidene complex.

2. Olefin metathesis catalyst in the form of a supported transition metal complex according to Claim 1, **characterized in that** z = 10.

3. Olefin metathesis catalyst in the form of a supported transition metal complex based on a silica gel matrix comprising structural units of the formula (II): 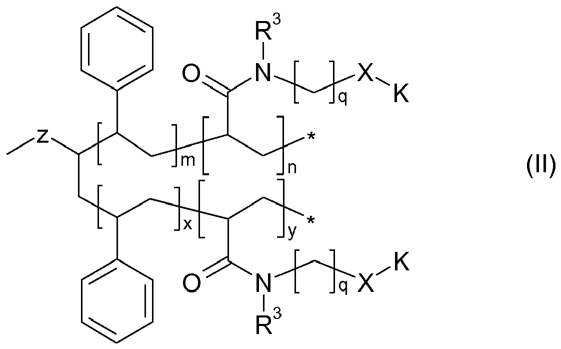 where
Z is a bond or a spacer;
m is from 1 to 5000;
n is from 1 to 5000;
x is from 1 to 5000;
y is from 1 to 5000;
X is a direct bond, oxygen, sulfur, -N(R¹)-, -C(=O)O-, -O(O=)C-, -N(R¹) (O=)C-, -C(=O)N(R¹)-, -O-CHR¹-O-, -OC(=O)N(R¹) N(R¹)C(=O)O-, >C(=O) or >C(=S);
R¹ is hydrogen or C₁-C₄-alkyl;
R³ is hydrogen or C₁-C₄-alkyl;
q is from 2 to 5,
K is a tungsten-, molybdenum- or ruthenium-alkylidene complex.

4. Olefin metathesis catalyst in the form of a supported transition metal complex according to Claim 3, **characterized in that** q = 3.

5. Olefin metathesis catalyst in the form of a supported transition metal complex according to either Claim 3 or 4, **characterized in that** R³ is methyl.

6. Olefin metathesis catalyst in the form of a supported transition metal complex according to any of Claims 1 to 5, **characterized in that** X is an amide bond, in particular -N(H)(O=)C-.

7. Olefin metathesis catalyst in the form of a supported transition metal complex according to any of Claims 1 to 6, **characterized in that** the transition metal complex is a molybdenum- or ruthenium-alkylidene complex.

8. Olefin metathesis catalyst in the form of a supported transition metal complex according to Claim 7, **characterized in that** the molybdenum- or ruthenium-alkylidene complex is derived from one of the following structures:
a structure of the formula (III): 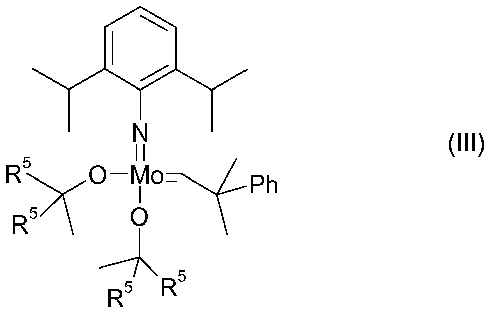 where
R⁵ is methyl or trifluoromethyl, or a structure of the formula (IVa): 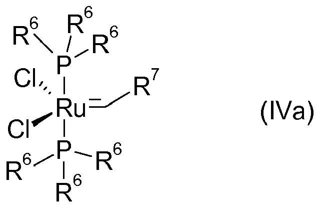 where
R⁶ is phenyl or cyclohexyl and
R⁷ is phenyl or CH=CPh₂, or
a structure of the formula (IVb): 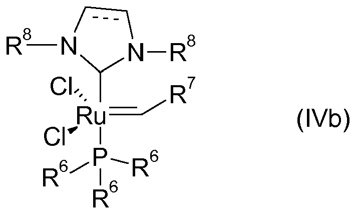 where
R⁶ is cyclohexyl;
R⁷ is phenyl; and
R⁸ is 2,4,6-trimethylphenyl (mesityl),
where "---" indicates an optional double bond, or a structure of the formula (IVc): 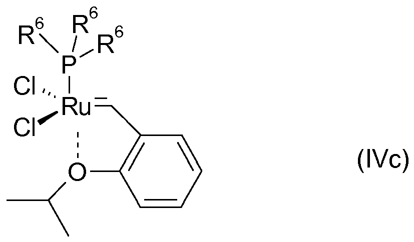 where
R⁶ is cyclohexyl, or
a structure of the formula (IVd): 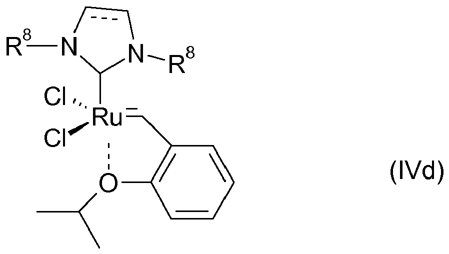 where
R⁸ is 2,4,6-trimethylphenyl (mesityl),
where "---" indicates an optional double bond.

9. Olefin metathesis catalyst in the form of a supported transition metal complex according to any of Claims 1 to 8, **characterized in that** K is a group of the formula (V): 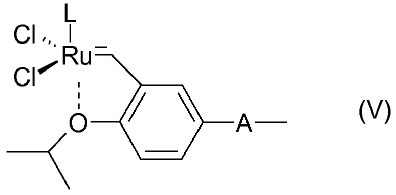 where
L is P(R⁶)₃ or 1,3-substituted 4,5-dihydroimidazol-2-ylidene, where R⁶ is cyclohexyl; and
A is C₁-C₁₀-alkylene or C₂-C₁₀-alkenylene bound to X.

10. Use of an olefin metathesis catalyst in the form of a supported transition metal complex according to any of Claims 1 to 9 in olefin metathesis.

11. Process for the transition metal-catalyzed olefin metathesis in supercritical carbon dioxide, **characterized in that** the catalyst is an olefin metathesis catalyst in the form of a supported transition metal complex according to any of Claims 1 to 9.

12. Process according to Claim 11, **characterized in that** the olefin metathesis is a ring-closing metathesis.

13. Process according to either of Claims 11 and 12, **characterized in that** the process is carried out continuously.

## Revendications

1. Catalyseur de métathèse d'oléfines sous la forme d'un complexe de métal de transition supporté à base d'une matrice de polystyrène, qui comprend des unités structurales de formule (Ia) : 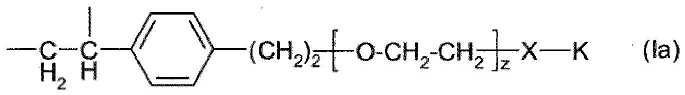 dans laquelle
z a la valeur d'un nombre de 3 à 20 ;
X représente une liaison directe, oxygène, soufre, - N(R¹)-, -C(=O)O-, -O(O=)C-, -N(R¹)(O=)C-, -C(=O)N(R¹)-, -O-CHR¹-O-, -OC(=O)N(R¹)-, -N(R¹)C (=O) O-, >C(=O) ou >C(=S);
R¹ représente hydrogène ou alkyle en C₁-C₄; et
K est un complexe de tungstène-, de molybdène- ou de ruthénium-alkylidène.

2. Catalyseur de métathèse d'oléfines sous la forme d'un complexe de métal de transition supporté selon la revendication 1, **caractérisé en ce que** z = 10.

3. Catalyseur de métathèse d'oléfines sous la forme d'un complexe de métal de transition supporté à base d'une matrice en gel de silice, qui comprend des unités structurales de formule (II) : 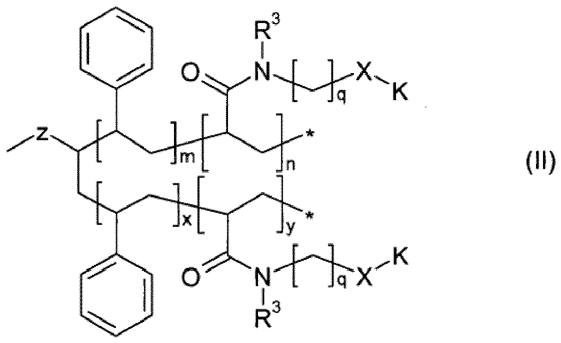 dans laquelle
Z représente une liaison ou un espaceur ;
m a la valeur d'un nombre de 1 à 5 000 ;
n a la valeur d'un nombre de 1 à 5 000 ;
x a la valeur d'un nombre de 1 à 5 000 ;
y a la valeur d'un nombre de 1 à 5 000 ;
X représente une liaison directe, oxygène, soufre, - N(R¹)-, -C(=O)O-, -O(O=)C-, -N(R¹)(O=)C-, -C(=O)N(R¹)-, -O-CHR¹-O-, -OC(=O)N(R¹)-, -N(R¹)C(=O)O-, >C(=O) ou >C(=S) ;
R¹ représente hydrogène ou alkyle en C₁-C₄ ;
R³ représente hydrogène ou alkyle en C₁-C₄ ;
q a la valeur d'un nombre de 2 à 5 ;
K est un complexe de tungstène-, de molybdène- ou de ruthénium-alkylidène.

4. Catalyseur de métathèse d'oléfines sous la forme d'un complexe de métal de transition supporté selon la revendication 3, **caractérisé en ce que** q = 3.

5. Catalyseur de métathèse d'oléfines sous la forme d'un complexe de métal de transition supporté selon l'une quelconque des revendications 3 à 4, **caractérisé en ce que** R³ représente méthyle.

6. Catalyseur de métathèse d'oléfines sous la forme d'un complexe de métal de transition supporté selon l'une quelconque des revendications 1 à 5, **caractérisé en ce que** X représente une liaison amide, notamment - N(H)(O=)C-.

7. Catalyseur de métathèse d'oléfines sous la forme d'un complexe de métal de transition supporté selon l'une quelconque des revendications 1 à 6, **caractérisé en ce que** le complexe de métal de transition est un complexe de molybdène- ou de ruthénium-alkylidène.

8. Catalyseur de métathèse d'oléfines sous la forme d'un complexe de métal de transition supporté selon la revendication 7, **caractérisé en ce que** le complexe de molybdène- ou de ruthénium-alkylidène dérive d'une des structures suivantes :
une structure de formule (III) : 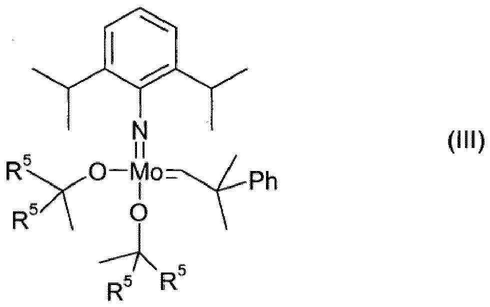 dans laquelle
R⁵ représente méthyle ou trifluorométhyle, ou
une structure de formule (IVa) : 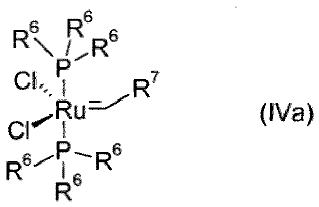
dans laquelle
R⁶ représente phényle ou cyclohexyle, et
R⁷ représente phényle ou CH=CPh₂, ou
une structure de formule (IVb) : 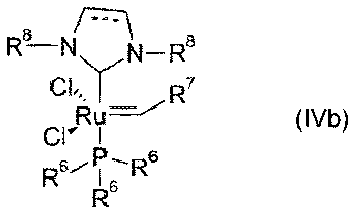 dans laquelle
R⁶ représente cyclohexyle ;
R⁷ représente phényle ; et
R⁸ représente 2,4,6-triméthylphényle (mésityle),
« --- » signifiant une double liaison optionnelle, ou
une structure de formule (IVc) : 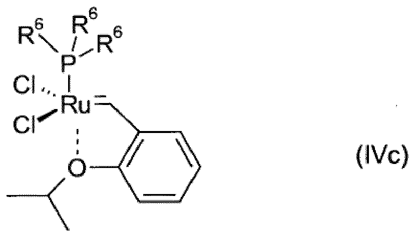 dans laquelle
R⁶ représente cyclohexyle, ou
une structure de formule (IVd) : 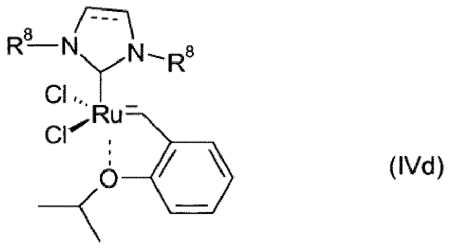 dans laquelle
R⁸ représente 2,4,6-triméthylphényle (mésityle),
« --- » signifiant une double liaison optionnelle.

9. Catalyseur de métathèse d'oléfines sous la forme d'un complexe de métal de transition supporté selon l'une quelconque des revendications 1 à 8, **caractérisé en ce que** K représente un groupe de formule (V) : 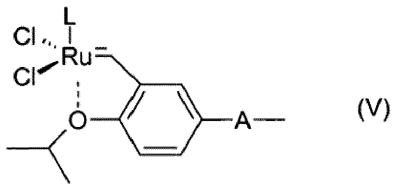 dans laquelle
L représente P(R⁶)₃ ou un 4,5-dihydroimidazol-2-ylidène 1,3-substitué, R⁶ représentant cyclohexyle ; et A représente un alkylène en C₁-C₁ₒ ou alcénylène en C₂-C₁₀ relié à X.

10. Utilisation d'un catalyseur de métathèse d'oléfines sous la forme d'un complexe de métal de transition supporté selon l'une quelconque des revendications 1 à 9 pour la métathèse d'oléfines.

11. Procédé de métathèse d'oléfines catalysée par un métal de transition dans du dioxyde de carbone surcritique, **caractérisé en ce que** le catalyseur est un catalyseur de métathèse d'oléfines sous la forme d'un complexe de métal de transition supporté selon l'une quelconque des revendications 1 à 9.

12. Procédé selon la revendication 11, **caractérisé en ce que** la métathèse d'oléfines est une métathèse par fermeture de cycle.

13. Procédé selon la revendication 11 ou 12, **caractérisé en ce que** le procédé est réalisé en continu.
